# EUROPÄISCHE PATENTSCHRIFT

(11) **EP 0 558 164 B1**
(45) Veröffentlichungstag und Bekanntmachung des Hinweises auf die Patenterteilung: **12.06.1996**
(21) Anmeldenummer: 93250034.1
(22) Anmeldetag: 27.01.1993
(51) Int. Cl.: B23K 26/00

(54) **Verfahren zum Schneiden mittels Laserstrahlung**
Procedure for cutting with laser radiation
Procédé pour couper avec rayonnement du laser

(30) Priorität: 27.01.1992 DE 4202487
(43) Veröffentlichungstag der Anmeldung: 01.09.1993
(73) Patentinhaber: OPTEC Gesellschaft für optische Technik mbH, D-57299 Burbach (DE)
(72) Erfinder: Müller, Gerhard J., Prof.Dr.-Ing., W-1000 Berlin 38 (DE); Giebel, Gerfried, Prof.Dr.med., W-6650 Homburg/Saar (DE)
(74) Vertreter: Christiansen, Henning, Dipl.-Ing.

(56) Entgegenhaltungen:
- DE-A- 1 565 144
- DE-A- 3 705 500
- DE-A- 3 829 728
- PATENT ABSTRACTS OF JAPAN vol. 14, no. 159 (M-956)(4102) 28. März 1990 & JP-A-20 20 681 (FUJITSU LTD) 24 Januar 1990
- PATENT ABSTRACTS OF JAPAN vol. 9, no. 276 (P-402)(1999) 2. November 1985 & JP-A-60 120 306 (NIPPON DENKI KK) 27 Juni 1985

## Beschreibung

Die Erfindung betrifft eine Vorrichtung zum Schneiden mittels Laserstrahlung.

Es ist bekannt, Laserstrahlung - ausgehend vom Laser-Generator - über Strahlführungssysteme bei der Materialbearbeitung zum Interaktionsort zu leiten und gegebenenfalls dort zur Erhöhung der Leistungs- bzw. Energiedichte mit abbildenden optischen Elementen zu fokussieren.

Dabei entspricht in aller Regel der Querschnitt des Fokus dem verkleinerten Bild der Strahltaille der Laserstrahlung im Resonator. Dieses Bild ist in der Praxis in etwa punktförmig. Strahlführungssysteme nach dem Stand der Technik sind dabei entweder als Mehrspiegelgelenkarme oder Lichtwellenleiter ausgebildet. Dabei weisen die Lichtwellenleiter, insbesondere im UV- oder IR-Bereich, meist noch technische Unzulänglichkeiten hinsichtlich der Übertragung höherer Energie- bzw. Leistungsdichten auf, so daß die gewünschten Energie- bzw. Leistungswerte, welche hinreichend hoch über der Einsatzschwelle des jeweiligen Prozesses liegen müssen, nicht sicher übertragen werden können.

Bei der Benutzung von Lasern im Spektralbereich von 150 nm bis 11 µm zum Schneiden von strukturierten Materialien, die dem schneidenden Laserstrahl unterschiedlichen Widerstand entgegesetzen - hier als "Composit-Material" bezeichnet - und hierbei insbesondere bei organischen Hartgeweben, besteht dabei zusätzlich noch das Problem, daß der fokussierte Strahl texturbedingt jeweils - bezogen auf gleiche Einwirkungszeiten - stark unterschiedliche Einschnittiefen zeigt.

Weiterhin ist es aus der deutschen Offenlegungsschrift Nr. 1 565 144 bekannt, bei der Materialbearbeitung mit Laserstrahlung eine Formung des Laserstrahls mit zylindrischen Linsen zu bewirken, um rechteckige Löcher zu bohren.

Aus DE-A-37 05 500 ist ein Verfahren zum Schneiden mittels Laserstrahlung bekannt, bei dem zur Steigerung der Schneideffizienz eine Fokussierung der Laserstrahlung längs einer quer zur Strahlungsrichtung verlaufenden Linie erfolgt, längs deren die Laserstrahlung in bezug auf das zu schneidende Material auch geführt wird.

Aus JP-A-2-20681 ist eine Vorrichtung zum Schneiden mittels Laserstrahlung bekannt, bei der eine anamorphotische Optik zur Fokussierung der Strahlung längs einer Linie vorgesehen ist. Die Strahlungsdichte im Fokussierungsgebiet ist hierbei im wesentlichen homogen, was für bestimmte Anwendungen nicht optimal ist.

Der Erfindung liegt die Aufgabe zugrunde, eine Vorrichtung anzugeben, bei der unter Beseitigung der eingangs genannten Nachteile unter verbesserter Energieausnutzung der Laserstrahlung eine Homogenisierung der Schnitteigenschaften erreicht werden kann.

Diese Aufgabe wird mit den Merkmalen der Ansprüche 1 bzw. 2 gelöst.

Die Erfindung schließt die Erkenntnis ein, daß durch die Ausrichtung der Laserstrahlung längs einer Linie und der damit gebildeten Linie von Interaktionen zwischen Laserstrahlung und zu bearbeitender Materialoberfläche eine Ausmittelung in der Weise eintritt, daß unter Einhaltung bzw. Überschreitung der erforderlichen (Mindest-) Schwellenenergie- bzw. Schwellenleistungdichte der Laserstrahlung sich sowohl Instabilitäten der Laserstrahlung als auch Inhomogenitäten des (Composit-)Materials in günstiger Weise ausmitteln lassen. Durch die Bündelung der Laserstrahlung entlang einer Linie (und gegebenenfalls entsprechende Führung entlang dieser Linie nach Art eines Skalpells) gelangen (nacheinander) Bereiche unterschiedlicher Widerstandsfähigkeit des Materials mit Bereichen unterschiedlicher Strahlungintensität in Kontakt, so daß sich eine höhere Schnittleistung erzielen läßt. Die gegenüber der ungebündelten Strahlung erhöhte Amplitude der örtlichen und zeitlichen Leistungsschwankungen ist dabei besser an den Schwankungsbereich der differentiellen örtlichen Veränderungen der Materialeigenschaften angepaßt, so daß der Materialabtrag vergleichmäßigt ist.

Insbesondere tritt auch durch die sukzessive Applikation von Laserenergie längs einer Linie bei einer schneidender (Hin- und Her-) Bewegung entsprechenden Handhabung des Gerätes eine Überlagerung der mit einzelnen Strahlungsanteilen längs der Linie erzielten Wirkung ein. Durch schneidenartige Konfiguration des Laserbündels wurde eine große Effektivität beim Materialabtrag mit großer lokaler Energiedichte erzielt, ohne daß dabei die Gesamterwärmung unzulässig große Werte erreichen würde.

In vorteilhafter Weiterbildung des Erfindungsgedankens wird bei einer Vorrichtung zur Durchführung des erfindungsgemäßen Verfahrens ein zweites anamorphotisches Bauelement zusätzlich zu dem in Anspruch 2 aufgeführten, jedoch relativ zu diesem um die optische Achse drehbar angeordnet. Dadurch kann in Abhängigkeit vom Verstellwinkel die Länge der Interaktionslinie in weiten Grenzen beliebig eingestellt werden, was besonders bei Änderung der Schnittrichtung mit kleinen Radien von Bedeutung ist.

Bei einer vorteilhaften Weiterbildung der erfindungsgemäßen Vorrichtung zur Durchführung von Präzisionsschnitten ist es vorgesehen, proportional zum Verstellwinkel der anamorphotischen Optik ein Signal abzuleiten, mit dessen Hilfe die Ausgangsleistung bzw. Energie des Lasers derart geändert werden kann, daß die Leistungs-(Energie-)dichte in der Interaktionszone immer einem gewünschten voreingestellten Wert entspricht.

In der Vorrichtung gemäß dem Anspruch 2 ist dem erstgenannten anamorphotischen Bauelement ein lineares Linsenarray nachgeschaltet, welches dazu dient, die Brennlinie in einzelne Brennpunkte längs einer Linie aufzulösen. Dies wird insbesondere dann angewendet, wenn zur Überschreitung der notwendigen Prozeßschwellen eine weitere Erhöhung der Energie-(Leistungs-)dichte in Betracht kommt.

Bei einer anderen Ansprägung des Erfindungsgedankens (Anspruch 1) kann die gestellte Aufgabe auch mit faseroptischen Bauelementen erreicht werden. Dabei wird die mit bekannten Mitteln erzeugte Laserstrahlung mittels Amplituden- oder Wellenfrontteilung in mehrere Einzelfasern eingekoppelt. Diese einzelnen Fasern nehmen dann zum distalen Ende hin eine in einer Ebene linienartig ausgerichtete Konfiguration an.

Bei dieser Erfindungsvariante ist die Linienlänge der nutzbaren Laserstrahlung durch eine veränderbare Neigung der Endbereiche der Fasern zueinander einstellbar.

Die mit der erfindungsgemäßen Laserschneidvorrichtung längs einer Linie fokussierte Laserstrahlung läßt sich insbesondere günstig durch einen Mehrspiegelgelenkarm zur Interaktionszone führen. Eine zur Fokussierung verwendete anamorphotische Optik besteht bevorzugt in einer Zylinderlinse. Die für die erfindungsgemäße Laserschneidvorrichtung benutzte Laserstrahlung weist insbesondere eine Wellenlänge zwischen 150 nm und 11 µm auf. Die variablen Pulslänge betragen dabei mindestens 10 ns.

Die Verkürzung der linienförmigen Fokussierung der Laserstrahlung läßt sich variieren durch eine weitere auf derselben optischen Achse drehbar vorgesehene Zylinderlinse.

Ein mit der Linsenfassung verbundener Positionsgeber gibt ein drehwinkelproportionales Signal ab, das zur Leistungs- bzw Energiedichteregelung nutzbar ist.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Vorrichtung zur Fokussierung der Strahlung längs einer Linie als Teildarstellung im Schnitt,
Figur 2 ein Variante der Vorrichtung gemäß Figur 1 mit drehbarer weiterer Zylinderlinse,
Figur 3 eine erste Ausführungsform gemäß der Erfindung mit zusätzlichen Linsen zur Fokussierung entlang einer Linie,
Figur 4 ein weiteres Ausführungsbeispiel gemäß der Erfindung, mit einer Aufteilung der einfallenden Laserstrahlung auf mehrere Lichtleiter,
Figuren 5 bis 5b eine Variante der Ausführung gemäß Figur 4 mit verschiedenen Detaildarstellungen,
Figur 6 ein weiteres Detail einer Ausführung gemäß Figuren 4 oder 5 zur Veränderung der Neigung der Lichtleitfasern untereinander sowie
Figur 7 eine Vorrichtung zur Veränderung der Länge des linienförmigen Querschnitts des mit einer der vorstehend dargestellten Ausführungsformen der Erfindung erzeugten Laserstrahlenbündels.

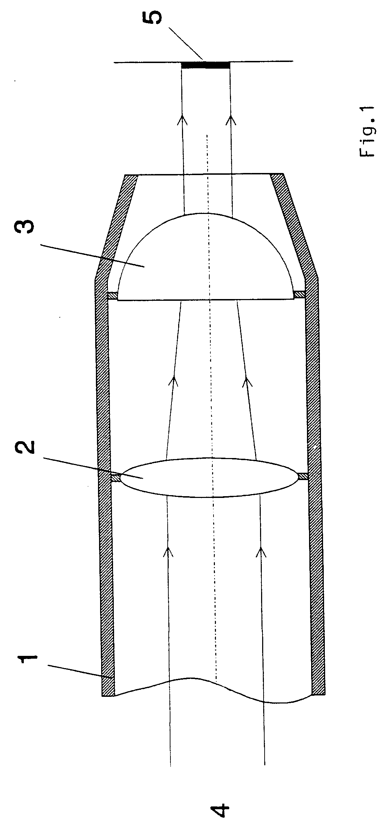

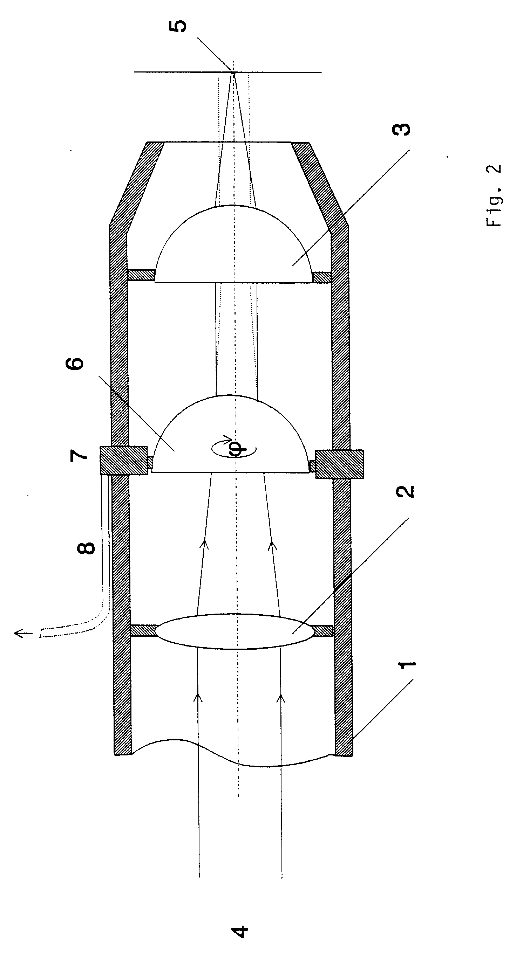

Die Vorrichtungen der Figuren 1 und 2 werden vom Schutzbereich der Patentansprüche nicht umfaßt.

Bei der in Figur 1 dargestellten Vorrichtung gelangt ein Laserstrahlenbündel 4 von einem nur in seinem distalen Bereich dargestellten Teil des Handstücks 1 über eine Sammellinse 2 zu einer Zylinderlinse 3, welche die anamorphotische Optik bildet. Durch die Zylinderlinse erfolgt eine Bündelung der Strahlung in Richtung auf das zu schneidende Composit-Material in der Weise, daß die Strahlung im Querschnitt ein rechteckiges bis linienförmiges Profil erhält. Mit dieser Vorrichtung lassen sich insbesondere Materialien mit inhomogener Struktur bearbeiten, so daß eine wesentliche Steigerung der Schneideffizienz bei Composit-Materialien erzielbar ist. Dabei erfolgt die Bewegung des Strahlenbündels in Bezug auf das zu schneidende Objekt bevorzugt in einer Hin- und Herbewegung in Richtung des größten Strahlungsquerschnitts nach Art der Führung eines Skalpells, so daß nacheinander unterschiedliche Teile des Strahlungsquerschnitts mit relativ dazu feststehenden Bereichen des zu schneidenden Materials in Wechselwirkung gelangen. Dabei mitteln sich sowohl örtliche Inhomogenitäten des Materials als auch solche der Laserstrahlung aus, so daß im Mittel eine gleichmäßige Schnittwirkung erzielt wird.

Die Laserstrahlung am distalen Ende eines (nicht dargestellten Spiegelgelenkarmes läßt sich dabei das Laserstrahlbündel derart auf eine Brennlinie konzentrieren, daß bei Überschreiten der Schwellenergie-(resp. Leistungs-) dichte die Laserstrahlung längs einer Interaktionslinie mit dem Material in Wechselwirkung tritt und damit Punkt-zu-Punkt-Instabilitäten der Laserstrahlung mit entsprechenden Punkt-zu-Punkt-Inhomogenitäten des Composit-Materials sehr gut ausgemittelt werden können.

Bei einer - in Figur 2 dargestellten - weiteren Vorrichtung entsprechend die mit übereinstimmenden Bezugszeichen versehenen Bauelemente in ihrer Funktion den anhand von Figur 1 beschriebenen. Zusätzlich zu dem ersten anamorphotischen Bauelement in Form der Zylinderlinse 3 ist ein weiteres anamorphotisches Bauelement 6 vorgesehen, welches zusätzlich um die (strichpunktiert dargestellte) optische Achse drehbar ist. Dadurch kann in Abhängigkeit vom Verstellwinkel die Länge der Interaktionslinie in weiten Grenzen beliebig eingestellt werden, was besonders bei Änderung der Schnittrichtung mit kleinen Radien von Bedeutung ist. Beim Einsatz der Vorrichtung zur Durchführung von Präzisionsschnitten wird proportional zum Verstellwinkel der anamorphotischen Optik ein Signal abgeleitet, mit dessen Hilfe die Ausgangsleistung bzw. Energie des Lasers derart geändert werden kann, daß die Leistungs-(Energie-)dichte in der Interaktionszone immer einem voreingestellten Wert entspricht.

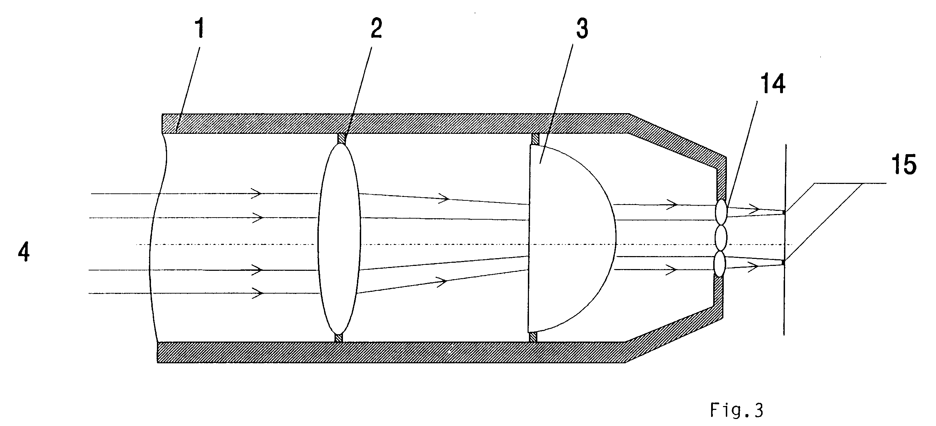

Bei einer ersten in Figur 3 dargestellten Ausführung der Erfindung ist - bei im übrigen unter Verwendung übereinstimmender Bezugszeichen einer der Vorrichtung nach Figur 1 entsprechenden Anordnung - dem erstgenannten anamorphotischen Bauelement 3 ein lineares Linsenarray 14 im optischen Strahlengang nachgeordnet, welches dazu dient, die Brennlinie in einzelne Brennpunkte längs einer Linie aufzulösen. Hiermit läßt sich eine weitere Energiekonzentration in einem Linienbereich erreichen, wenn sich bei der Überschreitung der notwendigen Prozeßschwellen eine weitere Erhöhung der Energie-(Leistungs-) dichte als notwendig erweisen sollte. Dies gilt insbesondere auch unter dem Gesichtspunkt der Bereitstellung von Strahlungsbereichen unterschiedlichen Energiegehalts bei Behandlung inhomegener Materialschichten.

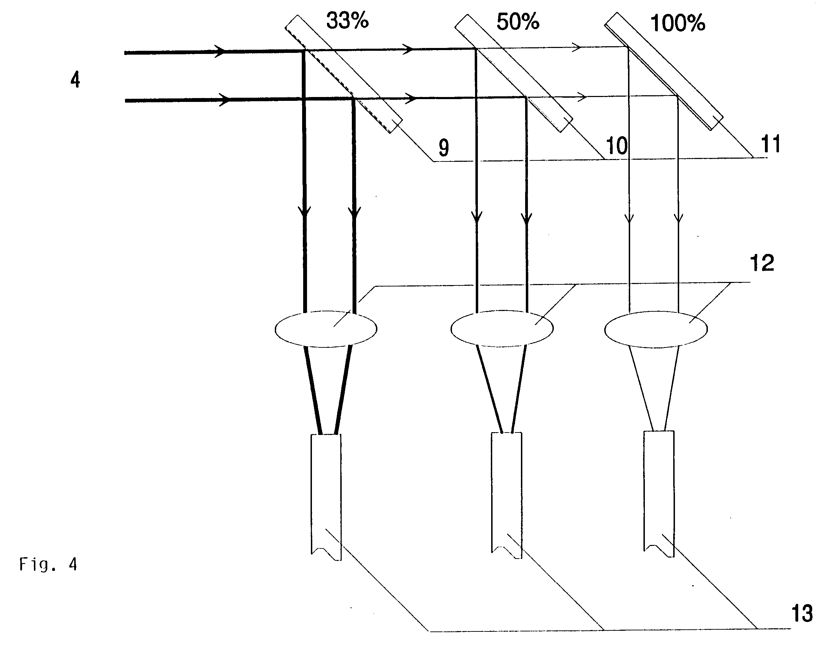

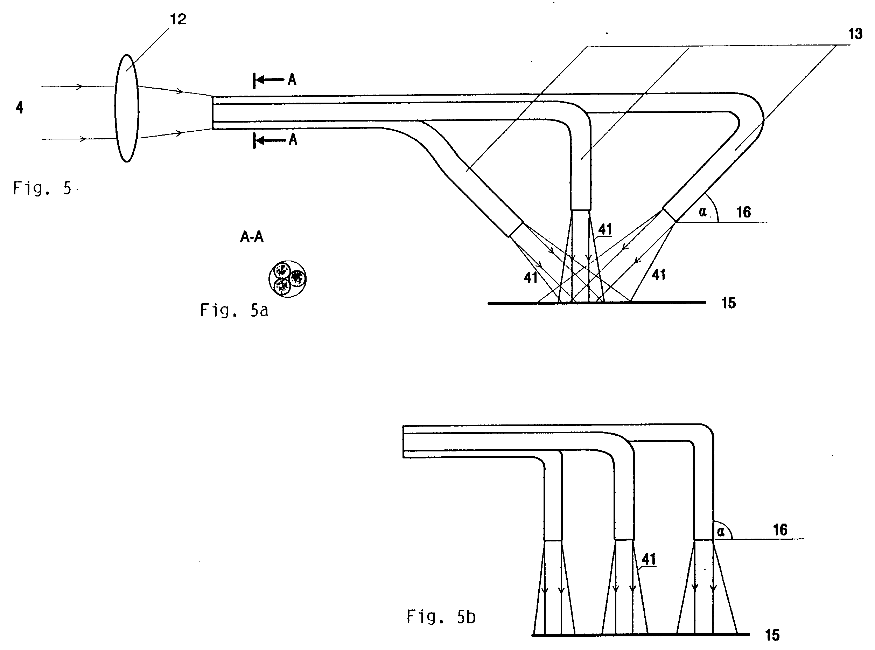

Bei einer weiteren Ausführungsform der Erfindung, wie sie in den Figuren 4 und 5 in zwei Varianten beschrieben ist, werden zur Lösung faseroptische Bauelemente verwendet, um eine linienförmige Erstreckung des Strahlungsquerschnitts zu erzielen.

Bei der Ausführung gemäß Figur 4 wird die einfallende Laserstrahlung mittels Amplitudenteilung durch Umlenkspiegel 9 bis 11 über zwischengeschaltete Sammellinsen auf die einzelnen Lichtleitfasern 13 verteilt, welche die so aufgeteilte Laserstrahlung zum distalen (Applikations-)Ende der Anordnung führen.

Eine Variation der Abmessungen des linienförmigen Querschnitts läßt sich bei dieser Ausführung ebenfalls durch eine drehbare Zylinderlinse erreichen, welche am distalen Austrittsende in einem linienförmigen Querschnittsbereich anzuordnen der Fasern anzuordnen wäre.

Bei der Ausführungsvariante gemäß Figur 5 wird die durch eine gemeinsame Sammellinse 12 einfallende Laserstrahlung durch Wellenfrontteilung in mehrere zu einem Bündel zusammengeführte Einzelfasern 13 eingeleitet. Der Querschnitt A-A gemäß Figur 5 ist in Figur 5a getrennt wiedergegeben und zeigt die Bündelanordnung der Einzelfasern, welche dazu dient, die von der Sammellinse 12 konzentrierte Strahlung möglichst vollständig in die Einzelfasern überzuleiten.

In Figur 5b sind die Einzelfaser in im Endbereich paralleler Ausrichtung dargestellt. Die Laserstrahlung fällt senkrecht auf die Materialoberfläche 15. Der zwischen einer parallel zur Materialoberfläche verlaufenden Geraden 16 und der Richtung der Fasern eingeschlossene Winkel α beträgt 90°. Diese einzelnen Fasern sind am distalen Ende in einer Ebene parallel angeordnet.

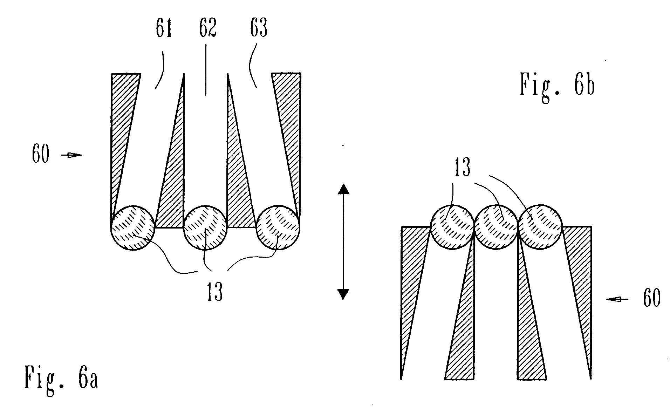

Gemäß einer Weiterbildung kann eine Veränderung der Linienlänge durch eine einstellbare Neigung der Fasern zueinander erreicht werden. Bei einer bevorzugten, in den Figuren 6a und b in zwei Positionen dargestellten, Weiterbildung ist die Länge der Ausdehnung der Strahlenbündels quer zur Strahlrichung mittels einer mit divergierende Führungen 61 bis 63 auf die im Querschnitt dargestellten Lichtleitfasern einwirkenden kammartigen Vorrichtung 60 einstellbar. Bei der in Figur 6a dargestellten Position weisen die Lichtleiter ihren weitesten Abstand auf, während sie in der Position gemäß Figur 6b dicht benachbart verlaufen, so daß die Länge der Quererstreckung des Laserbündels verringert ist. Die Lichtleitfasern sind dabei insbesondere in einiger Entfernung von der kammartigen Vorrichtung schwenkbar befestigt, so daß ihre relative Neigung veränderbar ist.

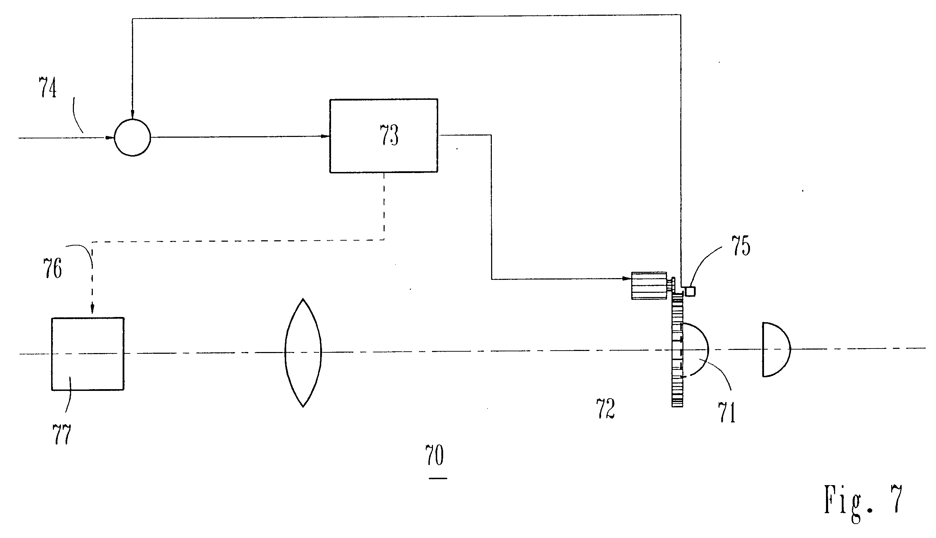

Bei einem in Figur 7 dargestellten Ausführungsbeispiel ist eine Schaltung zur selbständigen Nachsteuerung der Strahlungsenergie im Applikationsbereich für eine Anordnung der in Figur 2 dargestellten Art vorgesehen. Bei einer Strahlungsanordnung 70 ist die zweite Zylinderlinse 71 mittels eines mechanischen Antriebs rotierbar, der von einer Steuerschaltung 73 aktiviert wird. Durch Vorgabe eines Sollwertes 74 kann damit die Länge des Strahlungsquerschnitts eingestellt werden. Durch einen Positionssensor 75 und eine entsprechende Rückführung ist die Stellung der Zylinderlinse 71 exakt reproduzierbar.

Über eine gestrichelt dargestellte weitere Verbindung 76 zur Laserquelle 77 läßt sich weiterhin durch Beeinflussung der Dauer der Energieimpulse die Energiedichte im Applikationsraum zusätzlich - und damit insgesamt in weiten Grenzen - beeinflussen.

Liegt die erzielbare Bestrahlungsenergie unterhalb des gewünschten Sollwerts, so wird durch Verstellung der Zylinderlinse in Richtung einer Verkürzung der Linienlänge quer zur Strahlungsrichtung die Energiedichte am Objekt heraufgesetzt. Im anderen Fall erfolgt die Verstellung in umgekehrtem Richtungssinn. Auf diese Weise kann für verschiedene Materialien jeweils der optimale Energiewert empirisch ermittelt und durch externe Einstellung vorgegeben werden.

Durch die Regelungswirkung wird der betreffende Wert auch bei Instabilitäten der zugeführten Strahlung in einem weiten Bereich aufrechterhalten. Zusätzlich kann (wie erwähnt und mit den gestrichelten Pfeilen angedeutet) als weitere Größe zur Beeeinflussung der Laserenergie am Applikationsobjekt auch die Impulsdauer verändert werden. Größere Impulsdauern tragen dabei zur Heraufsetzung der abgegebenen Energie bei.

Die Erfindung beschränkt sich in ihrer Ausführung nicht auf das vorstehend angegebene bevorzugte Ausführungsbeispiel.

## Patentansprüche

1. Vorrichtung zum Schneiden mittels Laserstrahlung, bei der zur Übertragung der Laserstrahlung zu dem zu schneidenden Material (15) eine Mehrzahl von Lichtwellenleitern (13), insbesondere in Form von einzelnen Lichtleitfasern, vorgesehen ist, derart, daß durch Ausrichtung der Neigung der distalen Enden dieser Lichtwellenleiter längs einer geraden Linie eine Fokussierung längs einer Linie erfolgt, wobei die den einzelnen Lichtwellenleitern zugeführten Strahlungsanteile insbesondere aus einer einzigen Strahlungsquelle (4) durch Amplituden- oder Wellenfrontteilung abgeleitet werden.

2. Vorrichtung zum Schneiden mittels Laserstrahlung, mit einer anamorphotischen Optik (3) zur Fokussierung der Laserstrahlung längs einer Linie,
**dadurch gekennzeichnet,** daß
der anamorphotischen Optik (3) ein lineares Linsenarray (14) nachgeordnet ist, welches parallel zur Brennlinie ausgerichtet ist und die Brennlinie in einzelne Brennpunkte auflöst.

3. Vorrichtung nach Anspruch 2, **dadurch gekennzeichnet,** daß die anamorphotische Optik mindestens eine Zylinderlinse (3) aufweist.

4. Vorrichtung nach einem der vorangehenden Ansprüche, **dadurch gekennzeichnet,** daß zusätzlich zu der Anordnung zur Fokussierung (2, 3) längs einer quer zur Strahlrichtung verlaufenden Linie eine weitere, verstellbare Anordnung (6, 7, 8) vorgesehen ist, mit der die Länge der Linie bzw. der Grad der Fokussierung entlang der Linie veränderbar ist.

5. Vorrichtung nach Anspruch 4, **dadurch gekennzeichnet,** daß die verstellbare Anordnung aus einer, gegebenenfalls weiteren, Zylinderlinse (6) besteht, die relativ zu der Anordnung zur Fokussierung um eine in Strahlrichtung weisende Achse verdrehbar ist.

6. Vorrichtung nach Anspruch 1 oder einem der Ansprüche 3 bis 5, **dadurch gekennzeichnet,** daß eine verstellbare Anordnung (60) zu einer konformen Auslenkung der Enden der Lichtwellenleiter (13) in Richtung der Brennlinie im Sinne einer Strahlungsdispersion bzw. -konzentration vorgesehen ist.

7. Vorrichtung nach Anspruch 5 oder 6, **dadurch gekennzeichnet,** daß die verstellbare Anordnung (6, 7, 8) eine drehbare Halterung (7) aufweist.

8. Vorrichtung nach Anspruch 7, **dadurch gekennzeichnet,** daß an der drehbaren Halterung ein Positionsgeber (75) vorgesehen ist, der ein von der Winkelstellungsausrichtung der linienförmigen Fokussierung abhängiges, insbesondere zur Winkelstellung proportionales, Signal abgibt.

9. Vorrichtung nach Anspruch 7 oder 8, **dadurch gekennzeichnet,** daß die Halterung durch die Fassung (7) einer Linse (6) gebildet wird.

10. Vorrichtung nach Anspruch 8 oder 9, **dadurch gekennzeichnet,** daß eine Steuerungsvorrichtung (73) zur Beeinflussung der Energiedichte der Laserstrahlung in Abhängigkeit von dem drehwinkelabhängigen Signal vorgesehen ist.

11. Vorrichtung nach Anspruch 10, **dadurch gekennzeichnet,** daß eine Regelung in der Weise vorgesehen ist, daß die Energiedichte der Laserstrahlung unabhängig von der Brennlinienlänge im wesentlichen konstant bleibt.

12. Vorrichtung nach einem der vorangehenden Ansprüche, **dadurch gekennzeichnet,** daß die Laserstrahlung eine Wellenlänge zwischen im wesentlichen 150 nm und 11 µm aufweist.

13. Vorrichtung nach einem der vorangehenden Ansprüche, **dadurch gekennzeichnet,** daß die Impulsdauer der Laserstrahlung mindestens 10 ns beträgt.

## Claims

1. Apparatus for cutting using laser radiaton, wherein a plurality of light wave guides (13), particularly in the form of individual light conducting fibres, are provided for conveying the laser radiaton to the material (15) which is to be cut, in such a way that alignment of the inclination of the distal ends of these light wave guides along a straight line brings about focussing along a line, the radiaton components supplied to the individual light wave guides being derived particularly from a single radiation source (4) by amplitude or wave front division.

2. Apparatus for cutting using laser radiaton, having an anamorphotic optical device (3) for focussing the laser radiaton along a line, characterised in that a linear lens array (14) is mounted downstream of the anamorphotic optical device (3), said lens array (14) being aligned parallel to the focal line and resolving the focal line into individual foci.

3. Apparatus according to claim 2, characterised in that the anamorphotic optical device comprises at least one cylindrical lens (3).

4. Apparatus according to one of the preceding claims, characterised in that, in addition to the means for focussing (2, 3) along a line running transversely to the direction of the radiaton, there is another, adjustable arrangement (6, 7, 8) by means of which the length of the line or the degree of focussing along the line can be varied.

5. Apparatus according to claim 4, characterised in that the adjustable arrangement consists of a, possibly additional, cylindrical lens (6) which is rotatable, relative to the means for focussing, about an axis pointing in the direction of the radiaton.

6. Apparatus according to claim 1 or one of claims 3 to 5, characterised in that an adjustable arrangement (60) is provided for conformally deflecting the ends of the light wave guides (13) in the direction of the focal line in the sense of a dispersion or concentration of the radiaton.

7. Apparatus according to claim 5 or 6, characterised in that the adjustable arrangement (6, 7, 8) has a rotatable holder (7).

8. Apparatus according to claim 7, characterised in that a position indicator (75) is provided on the rotatable holder, emitting a signal which is dependent on the angular alignment of the linear focussing and, more particularly, proportional to the angular position.

9. Apparatus according to claim 7 or 8, characterised in that the holder is formed by the mount (7) of a lens (6).

10. Apparatus according to claim 8 or 9, characterised in that a control device (73) is provided for influencing the energy density of the laser radiaton as a function of the signal which is dependent on the angle of rotation.

11. Apparatus according to claim 10, characterised in that an adjustment is provided such that the energy density of the laser radiaton remains substantially constant irrespective of the length of the focal line.

12. Apparatus according to one of the preceding claims, characterised in that the laser radiation has a wavelength essentially between 150 nm and 11 µm.

13. Apparatus according to one of the preceding claims, characterised in that the pulse duration of the laser radiation is at least 10 ns.

## Revendications

1. Dispositif pour couper par rayonnement laser, dans lequel, pour transmettre le rayonnement laser au matériau (15) à couper, on a prévu une pluralité de guides d'ondes optiques (13) sous la forme de fibres optiques individuelles, dans un agencement tel que par l'orientation de l'inclinaison des extrémités distales de ces guides d'ondes optiques le long d'une ligne droite, le rayonnement est focalisé le long d'une ligne, les parties de rayonnement, amenées aux différents guides d'ondes optiques, étant obtenues en particulier d'une seule source de rayonnement (4) par division d'amplitude ou de front d'onde.

2. Dispositif pour couper par rayonnement laser, comprenant une optique anamorphosique (3) pour focaliser le rayonnement laser le long d'une ligne, caractérisé en ce que l'optique anamorphosique (3) est suivie d'un groupe linéaire de lentilles (14) qui est aligné parallèlement à la ligne focale et qui décompose la ligne locale en différents points focaux ou foyers.

3. Dispositif selon la revendication 2, caractérisé en ce que l'optique anamorphosique comporte au moins une lentille cylindrique (3).

4. Dispositif selon une des revendications précédentes, caractérisé en ce que, en plus du système de localisation (2, 3) le long d'une ligne orientée transversalement à la direction du rayonnement, on a prévu un système supplémentaire (6, 7, 8) qui est réglable et au moyen duquel la longueur de la ligne ou le degré de focalisation le long de la ligne est variable.

5. Dispositif selon la revendication 4, caractérisé en ce que le système réglable est formé d'une lentille cylindrique (6), constituant éventuellement une lentille cylindrique supplémentaire, qui peut être tournée par rapport au système de focalisation autour d'un axe orienté dans la direction du rayonnement.

6. Dispositif selon la revendication 1 ou l'une des revendications 3 à 5, caractérisé en ce qu'un système réglable (60) est prévu pour dévier de façon conforme les extrémités des fibres optiques (13) en direction de la ligne focale dans le sens d'une dispersion ou d'une concentration du rayonnement.

7. Dispositif selon la revendication 5 ou 6, caractérisé en ce que le système réglable (6, 7, 8) comporte un support (7) rotatif.

8. Dispositif selon la revendication 7, caractérisé en ce qu'un capteur de position (75) est prévu sur le support rotatif, capteur qui délivre un signal dépendant de la position angulaire coordonnée à la focalisation linéaire, en particulier un signal proportionnel à cette position angulaire.

9. Dispositif selon la revendication 7 ou 8, caractérisé en ce que le support est formé par la monture (7) d'une lentille (6).

10. Dispositif selon la revendication 8 ou 9, caractérisé en ce qu'un dispositif de commande (73) est prévu pour influencer la densité énergétique du rayonnement laser en fonction du signal dépendant de l'angle de rotation.

11. Dispositif selon la revendication 10, caractérisé en ce qu'une régulation est prévue pour faire en sorte que la densité énergétique du rayonnement laser reste essentiellement constante, indépendamment de la longueur de la ligne focale.

12. Dispositif selon une des revendications précédentes, caractérisé en ce que le rayonnement laser présente une longueur d'onde essentiellement comprise entre 150 nm et 11 µm.

13. Dispositif selon une des revendications précédentes, caractérisé en ce que la durée des impulsions du rayonnement laser est d'au moins 10 ns.
